(19) Europäisches Patentamt
European Patent Office
Office européen des brevets

(11) EP 3 071 616 B1

(12) **EUROPÄISCHE PATENTSCHRIFT**

(45) Veröffentlichungstag und Bekanntmachung des Hinweises auf die Patenterteilung:
**25.10.2017 Patentblatt 2017/43**

(21) Anmeldenummer: **14799487.5**

(22) Anmeldetag: **19.11.2014**

(51) Int Cl.:
***C08G 18/50*** *(2006.01)* ***C08G 18/71*** *(2006.01)*
***C08G 65/26*** *(2006.01)* ***C08G 65/333*** *(2006.01)*
***C08G 71/04*** *(2006.01)*

(86) Internationale Anmeldenummer:
**PCT/EP2014/074987**

(87) Internationale Veröffentlichungsnummer:
**WO 2015/075057 (28.05.2015 Gazette 2015/21)**

(54) **EINSATZ VON URETHAN-ALKOHOLEN ZUR HERSTELLUNG VON POLYETHERPOLYOLEN**
USE OF URETHANE ALCOHOLS FOR PRODUCING POLYETHER POLYOLS
UTILISATION D'ALCOOLS D'URÉTHANE POUR LA FABRICATION DE POLYÉTHERPOLYOLS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2013 EP 13194045**

(43) Veröffentlichungstag der Anmeldung:
**28.09.2016 Patentblatt 2016/39**

(73) Patentinhaber: **Covestro Deutschland AG**
**51373 Leverkusen (DE)**

(72) Erfinder:
- **HOFMANN, Jörg**
  **47800 Krefeld (DE)**
- **LAEMMERHOLD, Kai**
  **Shanghai, 200031 (CN)**
- **NEFZGER, Hartmut**
  **50259 Pulheim (DE)**
- **HEINZ, Monika**
  **51063 Köln (DE)**
- **KLESCZEWSI, Bert**
  **51069 Köln (DE)**
- **LORENZ, Klaus**
  **41539 Dormagen (DE)**

(74) Vertreter: **Levpat**
**c/o Covestro AG**
**Gebäude 4825**
**51365 Leverkusen (DE)**

(56) Entgegenhaltungen:
**EP-A1- 0 752 415** **US-A- 5 001 210**
**US-B2- 7 645 831**

- **John H.Clements: "Reactive applications of cyclic alkylene carbonates", Ind. Eng. Chem. Res., Bd. 42 18. Januar 2003 (2003-01-18), Seiten 663-674, XP002721719, Gefunden im Internet: URL:http://www.huntsman.com/performance_products/Media%20Library/a_MC348531CFA3EA9A2 E040EBCD2B6B7B06/Products_MC348531D0B9FA9A 2E040EBCD2B6B7B06/Carbonates_MC348531D1109 A9A2E040EBCD2B6B7B06/files/reactive_applications_of_cyclic_alkylene_carbonates.pdf [gefunden am 2015-03-09]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 071 616 B1

**Beschreibung**

Die vorliegende Erfindung betrifft ein Verfahren zur Herstellung von Polyetherpolyolen durch Anlagerung von Alkylenoxiden an H-funktionelle Starterverbindungen, dadurch gekennzeichnet, dass mindestens ein Urethan-Alkohol als H-funktionelle Starterverbindung eingesetzt wird. Weitere Gegenstände der Erfindung sind Polyetherpolyole enthaltend eine Urethangruppe, die Polyetherpolyole erhältlich nach dem erfindungsgemäßen Verfahren, die Verwendung der erfindungsgemäßen Polyetherpolyole zur Herstellung eines Polyurethanpolymers sowie die resultierenden Polyurethanpolymere.

Die Herstellung von Polyethercarbonatpolyolen durch katalytische Umsetzung von Alkylenoxiden (Epoxiden) und Kohlendioxid in Anwesenheit von H-funktionellen Startersubstanzen ("Starter") wird seit mehr als 40 Jahren intensiv untersucht (z. B. Inoue et al, Copolymerization of Carbon Dioxide and Epoxide with Organometallic Compounds; Die Makromolekulare Chemie 130, 210-220, 1969). Diese Reaktion ist in Schema (I) schematisch dargestellt, wobei R für einen organischen Rest wie Alkyl, Alkylaryl oder Aryl steht, der jeweils auch Heteroatome wie beispielsweise O, S, Si usw. enthalten kann, und wobei e, f, g und h für eine ganzzahlige Zahl stehen, und wobei das hier im Schema (I) gezeigte Produkt für das Polyethercarbonatpolyol lediglich so verstanden werden soll, dass sich Blöcke mit der gezeigten Struktur im erhaltenen Polyethercarbonatpolyol prinzipiell wiederfinden können, die Reihenfolge, Anzahl und Länge der Blöcke sowie die OH-Funktionalität des Starters aber variieren kann und nicht auf das in Schema (I) gezeigte Polyethercarbonatpolyol beschränkt ist. Diese Reaktion (siehe Schema (I)) ist ökologisch sehr vorteilhaft, da diese Reaktion die Umsetzung eines Treibhausgases wie $CO_2$ zu einem Polymer darstellt. Als weiteres Produkt, eigentlich Nebenprodukt, entsteht das in Schema (I) gezeigte cyclische Carbonat (beispielsweise für R = $CH_3$ Propylencarbonat, im Folgenden auch als cPC bezeichnet, oder für R = H Ethylencarbonat, im Folgenden auch als cEC bezeichnet).

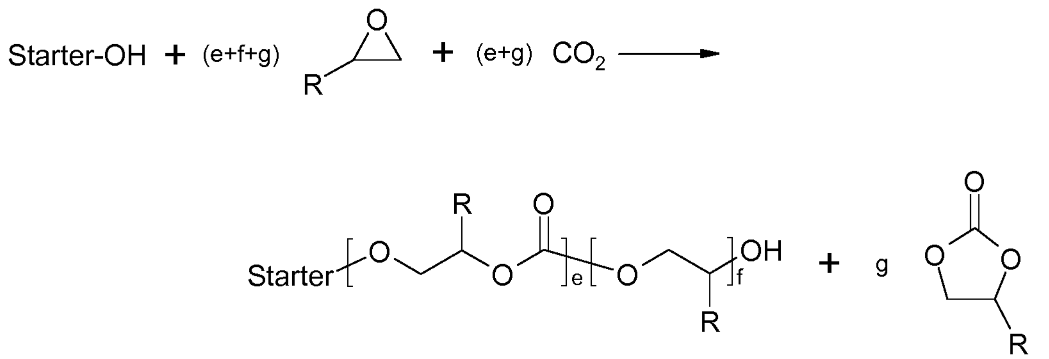

(I)

US 3,829,505 bzw. DE 1 595 759 beschreiben die Möglichkeit, OH-funktionelle Starterverbindungen im Überschuss mit aromatischen Polyisocyanaten umzusetzen, um auf diese Weise zu OH-Gruppen enthaltenden Polyurethanpolyolen mit mindestens 2 Urethangruppen zu gelangen, die als Starteroligomere für die DMC-Katalyse verwendet werden können.

US 3,654,224 beschreibt die Möglichkeit, Amide, inbesondere aromatische Amide wie z.B. Benzamid, als Starterverbindung für die DMC-Katalyse einzusetzen. US 5 001 210 offenbart im Beispiel 9 die Herstellung eines Polyether-Urethan-Diols aus Jeffamine ED600 und Ethylencarbonat. Außerdem wird das Polyether-Urethan-Diol zur Herstellung PUR verwendet. Die der vorliegenden Erfindung zu Grunde liegende Aufgabe bestand deshalb darin, das als Nebenprodukt anfallende cyclische Carbonat für die Herstellung von Polyetherpolyolen zu verwerten. Vorzugsweise sollen die so erhältlichen Polyetherpolyole für die Herstellung von Polyurethanen, insbesondere von Polyurethan-Weichschaumstoffen geeignet sein.

Diese Aufgabe wird erfindungsgemäß gelöst durch ein Verfahren zur Herstellung von Polyetherpolyolen durch Anlagerung von Alkylenoxiden an H-funktionelle Starterverbindungen, dadurch gekennzeichnet, dass mindestens ein Urethan-Alkohol gemäß Formel (II)

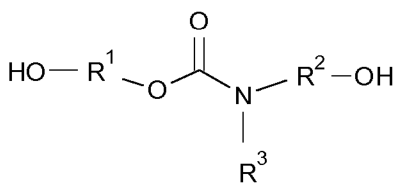

(II)

wobei

- $R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
- $R^2$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$, und
- $R^3$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei R1 bis R3 identisch oder voneinander verschieden sein können, als H-funktionelle Starterverbindung eingesetzt wird.

Die Verwendung des Worts *ein* im Zusammenhang mit zählbaren Größen ist hierbei und im Folgenden nur dann als Zahlwort zu verstehen, wenn dies aus dem Zusammenhang hervorgeht (bspw. durch die Formulierung *"genau ein"*). Ansonsten umfassen Ausdrücke wie "ein Alkylenoxid", "ein Urethan-Alkohol" etc. immer auch solche Ausführungsformen, in denen zwei oder mehr Alkylenoxide, zwei oder mehr Urethan-Alkohole etc. eingesetzt werden.

Nachfolgend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

Bevorzugt sind die Urethan-Alkohole der Formel (II) erhältlich durch die Umsetzung von cyclischen Carbonaten mit Aminoalkoholen. Unter Aminoalkoholen werden im Sinne der Erfindung Verbindungen verstanden, die mindestens eine Amino- und mindestens eine OH-Gruppe aufweisen. Als cyclische Carbonate werden dabei bevorzugt solche eingesetzt, die bei der Copolymerisation von Alkylenoxiden mit $CO_2$ als Nebenprodukte gebildet werden, beispielsweise sind dies Propylencarbonat (cPC) und Ethylencarbonat (cEC).

Als Aminoalkohole werden bevorzugt solche eingesetzt, die primäre oder sekundäre Aminogruppen, bevorzugt primäre Aminogruppen aufweisen, besonders bevorzugt wird als Aminoalkohol Ethanolamin oder Isopropanolamin eingesetzt.

Bevorzugt sind die Urethan-Alkohole der Formel (II) erhältlich durch Umsetzung von Propylencarbonat und/oder Ethylencarbonat mit Aminoalkoholen gemäß Formel (III),

$$HN(R^3)\text{-}R^2\text{-}OH \qquad (III)$$

wobei $R^2$ und $R^3$ die oben genannte Bedeutung haben.

Besonders bevorzugt sind die Urethan-Alkohole der Formel (II) erhältlich durch Umsetzung von Propylencarbonat und/oder Ethylencarbonat mit mindestens einem Amin ausgewählt aus der Gruppe bestehend aus Ethanolamin, Diethanolamin, (N-Methyl)-Ethanolamin, Isopropanolamin, Diisopropanolamin und Propanolamin.

Die Umsetzung der cyclischen Carbonate mit den Aminoalkoholen erfolgt bevorzugt bei 40 bis 80 °C, besonders bevorzugt bei 55 bis 65 °C. Die Reaktionszeit liegt bevorzugt bei 5 bis 40 h, besonders bevorzugt bei 10 bis 30 h.

In einer besonders vorteilhaften Ausführungsform wird das cyclische Carbonat im Überschuss eingesetzt. Bevorzugt beträgt das molare Verhältnis von cyclischem Carbonat zu Aminoalkohol 1,05 bis 3, besonders bevorzugt von 1,1 bis 2, ganz besonders bevorzugt von 1,2 bis 1,6. Das überschüssige cyclische Carbonat kann entweder direkt nach der Synthese des Urethan-Alkohols durch z.B. Dünnschichtverdampfung entfernt werden, oder kann im Urethan-Alkohol belassen und bei der Polyetherpolyolherstellung mit eingesetzt werden. Im zweiten genannten Fall wird das überschüssige cyclische Carbonat nach der Polyetherpolyolherstellung aus dem Produkt entfernt.

Neben den Urethan-Alkoholen können im erfindungsgemäßen Verfahren zusätzlich auch H-funktionelle Starterverbindungen ohne Urethangruppen eingesetzt werden, die nachfolgend beschrieben werden. Als geeignete H-funktionelle Startersubstanz ("Starter") können Verbindungen mit für die Alkoxylierung aktiven H-Atomen eingesetzt werden, die eine Molmasse von 18 bis 4500 g/mol, bevorzugt von 62 bis 500 g/mol und besonders bevorzugt von 62 bis 182 g/mol aufweisen. Die Fähigkeit zur Verwendung eines Starters mit einer niedrigen Molmasse ist ein deutlicher Vorteil gegenüber der Verwendung von oligomeren Startern, die mittels einer vorherigen Oxyalkylierung hergestellt sind. Insbesondere wird eine Wirtschaftlichkeit erreicht, die durch das Weglassen eines getrennten Oxyalkylierungsverfahrens ermöglicht wird.

Für die Alkoxylierung aktive Gruppen mit aktiven H-Atomen sind beispielsweise -OH, $-NH_2$ (primäre Amine), -NH- (sekundäre Amine), -SH und $-CO_2H$, bevorzugt sind -OH und $-NH_2$, besonders bevorzugt ist -OH. Als H-funktionelle Startersubstanz wird beispielsweise eine oder mehrere Verbindungen ausgewählt aus der Gruppe bestehend aus ein- oder mehrwertigen Alkoholen, mehrwertigen Aminen, mehrwertigen Thiolen, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyethylenimine, Polyetheramine, Polytetrahydrofurane (z. B. PolyTHF® der BASF), Polytetrahydrofuranamine, Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und $C_1$-$C_{24}$ Alkyl-Fettsäureester, die im Mittel mindestens 2 OH-Gruppen pro Molekül enthalten, eingesetzt. Beispielhaft handelt es sich bei den $C_1$-$C_{24}$ Alkyl-Fettsäureester, die im Mittel mindestens 2 OH-Gruppen pro Molekül enthalten, um Handelsprodukte wie Lupranol Balance® (Fa. BASF AG), Merginol®-Typen (Fa. Hobum Oleochemicals GmbH), Sovermol®-Typen (Fa. Cognis Deutschland GmbH & Co. KG) und Soyol®TM-Typen (Fa. USSC Co.).

Als monofunktionelle Startersubstanzen können Alkohole, Amine, Thiole und Carbonsäuren eingesetzt werden. Als monofunktionelle Alkohole können Verwendung finden: Methanol, Ethanol, 1-Propanol, 2-Propanol, 1-Butanol, 2-Butanol, tert-Butanol, 3-Buten-1-ol, 3-Butin-1-ol, 2-Methyl-3-buten-2-ol, 2-Methyl-3-butin-2-ol, Propagylalkohol, 2-Methyl-2-propanol, 1-tert-Butoxy-2-propanol, 1-Pentanol, 2-Pentanol, 3-Pentanol, 1-Hexanol, 2-Hexanol, 3-Hexanol, 1-Heptanol, 2-Heptanol, 3-Heptanol, 1-Octanol, 2-Octanol, 3-Octanol, 4-Octanol, Phenol, 2-Hydroxybiphenyl, 3-Hydroxybiphenyl, 4-Hydroxybiphenyl, 2-Hydroxypyridin, 3-Hydroxypyridin, 4-Hydroxypyridin. Als monofunktionelle Amine kommen in Frage: Butylamin, tert-Butylamin, Pentylamin, Hexylamin, Anilin, Aziridin, Pyrrolidin, Piperidin, Morpholin. Als mono funktionelle Thiole können verwendet werden: Ethanthiol, 1-Propanthiol, 2-Propanthiol, 1-Butanthiol, 3-Methyl-1-butanthiol, 2-Buten-1-thiol, Thiophenol. Als monofunktionelle Carbonsäuren seien genannt: Ameisensäure, Essigsäure, Propionsäure, Buttersäure, Fettsäuren wie Stearinsäure, Palmitinsäure, Ölsäure, Linolsäure, Linolensäure, Benzoesäure, Acrylsäure.

Als H-funktionelle Startersubstanzen geeignete mehrwertige Alkohole sind beispielsweise zweiwertige Alkohole (wie beispielsweise Ethylenglykol, Diethylenglykol, Propylenglykol, Dipropylenglykol, 1,3-Propandiol, 1,4-Butandiol, 1,4-Butendiol, 1,4-Butindiol, Neopentylglykol, 1,5-Pentandiol, Methylpentandiole (wie beispielweise 3-Methyl-1,5-pentandiol), 1,6-Hexandiol, 1,8-Octandiol, 1,10-Decandiol, 1,12-Dodecandiol, Bis-(hydroxymethyl)-cyclohexane (wie beispielweise 1,4-Bis-(hydroxymethyl)cyclohexan), Triethylenglykol, Tetraethylenglykol, Polyethylenglykole, Dipropylenglykol, Tripropylenglykol, Polypropylenglykole, Dibutylenglykol und Polybutylenglykole); dreiwertige Alkohole (wie beispielsweise Trimethylolpropan, Glycerin, Trishydroxyethylisocyanurat, Rizinusöl); vierwertige Alkohole (wie beispielsweise Pentaerythrit); Polyalkohole (wie beispielsweise Sorbit, Hexit, Saccharose, Stärke, Stärkehydrolysate, Cellulose, Cellulosehydrolysate, hydroxyfunktionalisierte Fette und Öle, insbesondere Rizinusöl), sowie alle Modifizierungsprodukte dieser zuvorgenannten Alkohole mit unterschiedlichen Mengen an ε-Caprolacton.

Die H-funktionellen Startersubstanzen können auch aus der Substanzklasse der Polyetherpolyole ausgewählt sein, die ein Molekulargewicht $M_n$ im Bereich von 18 bis 4500 g/mol und eine Funktionalität von 2 bis 3 aufweisen. Bevorzugt sind Polyetherpolyole, die aus sich wiederholenden Ethylenoxid- und Propylenoxideinheiten aufgebaut sind, bevorzugt mit einem Anteil von 35 bis 100% Propylenoxideinheiten, besonders bevorzugt mit einem Anteil von 50 bis 100% Propylenoxideinheiten. Hierbei kann es sich um statistische Copolymere, Gradienten-Copolymere, alternierende oder Blockcopolymere aus Ethylenoxid und Propylenoxid handeln. Insbesondere werden Polyetherpolyole, die nach dem hier beschriebenen erfindungsgemäßen Verfahren erhältlich sind, eingesetzt. Diese als H-funktionelle Startersubstanzen eingesetzten Polyetherpolyole werden hierzu in einem separaten Reaktionsschritt zuvor hergestellt

Die H-funktionellen Startersubstanzen können auch aus der Substanzklasse der Polyesterpolyole ausgewählt sein. Als Polyesterpolyole werden mindestens difunktionelle Polyester eingesetzt. Bevorzugt bestehen Polyesterpolyole aus alternierenden Säure- und Alkoholeinheiten. Als Säurekomponenten werden z. B. Bernsteinsäure, Maleinsäure, Maleinsäureanhydrid, Adipinsäure, Phthalsäureanhydrid, Phthalsäure, Isophthalsäure, Terephthalsäure, Tetrahydrophtalsäure, Tetrahydrophthalsäureanhydrid, Hexahydrophthalsäureanhydrid oder Gemische aus den genannten Säuren und/oder Anhydriden eingesetzt. Als Alkoholkomponenten werden z. B. Ethandiol, 1,2-Propandiol, 1,3-Propandiol, 1,4-Butandiol, 1,5-Pentandiol, Neopentylglykol, 1,6-Hexandiol, 1,4-Bis-(hydroxymethyl)-cyclohexan, Diethylenglykol, Dipropylenglykol, Trimethylolpropan, Glycerin, Pentaerythrit oder Gemische aus den genannten Alkoholen verwendet. Werden als Alkoholkomponente zweiwertige oder mehrwertige Polyetherpolyole eingesetzt, so erhält man Polyesteretherpolyole, die ebenfalls als Startersubstanzen zur Herstellung der Polyethercarbonatpolyole dienen können.

Des Weiteren können als H-funktionelle Startersubstanzen Polycarbonatdiole eingesetzt werden, die beispielsweise durch Umsetzung von Phosgen, Dimethylcarbonat, Diethylcarbonat oder Diphenylcarbonat und difunktionellen Alkoholen oder Polyesterpolyolen oder Polyetherpolyolen hergestellt werden. Beispiele zu Polycarbonaten finden sich z. B. in der EP-A 1359177.

In einer weiteren Ausführungsform der Erfindung können Polyethercarbonatpolyole als H-funktionelle Startersubstanzen eingesetzt werden.

Die H-funktionellen Startersubstanzen weisen im Allgemeinen eine Funktionalität (d.h. Anzahl an für die Polymerisation aktiven H-Atomen pro Molekül) von 1 bis 8, bevorzugt von 2 oder 3 auf. Die H-funktionellen Startersubstanzen werden entweder einzeln oder als Gemisch aus mindestens zwei H-funktionellen Startersubstanzen eingesetzt.

Besonders bevorzugt handelt es sich bei den H-funktionellen Startersubstanzen um eine oder mehrere Verbindungen ausgewählt aus der Gruppe bestehend aus Ethylenglykol, Propylenglykol, 1,3-Propandiol, 1,3-Butandiol, 1,4-Butandiol, 1,5-Pentandiol, 2-Methylpropan-1,3-diol, Neopentylglykol, 1,6-Hexandiol, 1,8-Octandiol, Diethylenglykol, Dipropylenglykol, Glycerin, Trimethylolpropan, Pentaerythrit, Sorbitol und Polyetherpolyole mit einem Molekulargewicht Mn im Bereich von 150 bis 4500 g/mol und einer Funktionalität von 2 bis 3.

Ein weiterer Gegenstand der Erfindung sind Polyetherpolyole enthaltend eine Struktureinheit der Formel (IV),

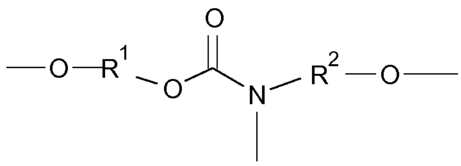

(IV)

wobei $R^1$ und $R^2$ die oben genannte Bedeutung haben. Vorzugsweise enthalten die erfindungsgemäßen Polyetherpolyole genau eine einzige Struktureinheit der Formel (IV) pro Polyetherpolyol-Molekül.

Die erfindungsgemäßen Polyetherpolyole weisen bevorzugt eine OH-Zahl von 3 bis 400 mg KOH /g, besonders bevorzugt 10 bis 200 mg KOH/g auf.

Des Weiteren weisen die erfindungsgemäßen Polyetherpolyole eine Funktionalität von 2,0 bis 3,0, bevorzugt von 2,5 bis 2,95 auf.

Ein weiterer Gegenstand der vorliegenden Erfindung ist ein Verfahren zur Herstellung von Polyetherpolyolen durch Anlagerung von Alkylenoxiden an H-funktionelle Starterverbindungen, dadurch gekennzeichnet, dass mindestens ein Urethan-Alkohol gemäß Formel (II) als H-funktionelle Starterverbindung eingesetzt wird und die Anlagerung in Gegenwart mindestens eines Doppelmetallcyanid-Katalysators (auch als DMC-Katalysator bezeichnet) erfolgt.

Für das erfindungsgemäße Verfahren geeignete DMC-Katalysatoren sind im Prinzip aus dem Stand der Technik bekannt (siehe z.B. US-A-3,404,109, US-A-3,829,505, US-A-3,941,849 und US-A-5,158,922). DMC-Katalysatoren, die z. B. in US-A-5,470,813, EP-A-0 700 949, EP-A-0 743 093, EP-A-0 761 708, WO 97/40086, WO 98/16310 und WO 00/47649 beschrieben sind, besitzen eine sehr hohe Aktivität in der Polymerisation von Alkylenoxiden und ggf. der Co-Polymerisation von Alkylenoxiden mit geeigneten Co-Monomeren wie beispielsweise Lactonen, cyclischen Carbonsäureanhydriden, Lactiden, cyclischen Carbonaten oder Kohlendioxid und ermöglichen die Herstellung von polymeren Polyolen bei sehr geringen Katalysatorkonzentrationen (25 ppm oder weniger), so dass eine Abtrennung des Katalysators aus dem fertigen Produkt im Allgemeinen nicht mehr erforderlich ist. Ein typisches Beispiel sind die in EP-A-0 700 949 beschriebenen hochaktiven DMC-Katalysatoren, die neben einer Doppelmetallcyanid-Verbindung (z. B. Zinkhexacyanocobaltat(III)) und einem organischen Komplexliganden (z.B. tert.-Butanol) noch einen Polyether mit einem zahlenmittlerem Molekulargewicht größer als 500 g/mol enthalten.

Es ist auch möglich, die in WO 2011/144523 offenbarten alkalischen DMC-Katalysatoren einzusetzen.

Zur Herstellung der Doppelmetallcyanid-Verbindungen geeignete cyanidfreie Metallsalze besitzen bevorzugt die allgemeine Formel (V),

$$M(X)_n \qquad (V)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$,
X steht für ein oder mehrere (d. h.verschiedene) Anionen, vorzugsweise ein Anion ausgewählt aus der Gruppe der Halogenide (d. h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
n ist 1, wenn X = Sulfat, Carbonat oder Oxalat ist und
n ist 2, wenn X = Halogenid, Hydroxid, Cyanat, Thiocyanat, Isocyanat, Isothiocyanat oder Nitrat ist;
oder geeignete cyanidfreie Metallsalze besitzen die allgemeine Formel (VI),

$$M_r(X)_3 \qquad (VI)$$

wobei

M ausgewählt ist aus den Metallkationen $Fe^{3+}$, $Al^{3+}$ und $Cr^{3+}$,
X steht für ein oder mehrere (d. h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
r ist 2, wenn X = Sulfat, Carbonat oder Oxalate ist und
r ist 1, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (VII),

$$M(X)_s \qquad \text{(VII)}$$

wobei

M ausgewählt ist aus den Metallkationen $Mo^{4+}$, $V^{4+}$ und $W^{4+}$
X steht für oder mehrere (d. h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
s ist 2, wenn X = Sulfat, Carbonat oder Oxalat ist und
s ist 4, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (VIII),

$$M(X)_t \qquad \text{(VIII)}$$

wobei

M ausgewählt ist aus den Metallkationen $Mo^{6+}$ und $W^{6+}$
X steht für ein oder mehrere (d. h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
t ist 3, wenn X = Sulfat, Carbonat oder Oxalat ist und
t ist 6, wenn X = Halogenid, Hydroxid, Cyanat, Thiocyanat, Isocyanat, Isothiocyanat, Carboxylat oder Nitrat ist.

Beispiele geeigneter cyanidfreier Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel-(II)nitrat. Es können auch Mischungen verschiedener Metallsalze eingesetzt werden.
Zur Herstellung der Doppelmetallcyanid-Verbindungen geeignete Metallcyanidsalze besitzen bevorzugt die allgemeine Formel (IX)

$$(Y)_a M'(CN)_b (A)_c \qquad \text{(IX)}$$

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),
Y ausgewählt ist aus einem oder mehreren Metallkationen der Gruppe bestehend aus Alkalimetall (d.h. $Li^+$, $Na^+$, $K^+$, $Rb^+$, $Cs^+$) und Erdalkalimetall (d.h. $Be^{2+}$, $Ca^{2+}$, $Mg^{2+}$, $Sr^{2+}$, $Ba^{2+}$),
A ausgewählt ist aus einem oder mehreren Anionen der Gruppe bestehend aus Halogeniden (d..h. Fluorid, Chlorid, Bromid, Iodid), Hydroxid, Sulfat, Carbonat, Cyanat, Thiocyanat, Isocyanat, Isothiocyanat, Carboxylat, Oxalat oder Nitrat und
a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).
Bevorzugte Doppelmetallcyanid-Verbindungen, die in den DMC-Katalysatoren enthalten sind, sind Verbindungen der allgemeinen Formel (X)

$$M_x[M'_{x'}(CN)_y]_z \qquad \text{(X),}$$

worin M wie in Formel (V) bis (VIII) und

M' wie in Formel (IX) definiert ist, und
x, x', y und z sind ganzzahlig und so gewählt, dass die Elektronenneutralität der Doppelmetallcyanidverbindung gegeben ist.

Vorzugsweise ist
x = 3, x' = 1,y = 6 und z = 2,
M = Zn(II), Fe(II), Co(II) oder Ni(II) und
M' = Co(III), Fe(III), Cr(III) oder Ir(III).

Beispiele geeigneter Doppelmetallcyanidverbindungen sind Zinkhexacyanocobaltat(III), Zinkhexacyanoiridat(III), Zinkhexacyanoferrat(III) und Cobalt(II)hexacyanocobaltat(III). Weitere Beispiele geeigneter Doppelmetallcyanid-Verbindungen sind z.B. US-A 5158922 (Spalte 8, Zeilen 29 - 66) zu entnehmen. Besonders bevorzugt verwendet wird Zinkhexacyanocobaltat(III).

Die bei der Herstellung der DMC-Katalysatoren zugesetzten organischen Komplexliganden sind beispielsweise in US-A-5,158,922 (siehe insbesondere Spalte 6, Zeilen 9 bis 65), US-A-3,404,109, US-A-3,829,505, US-A-3,941,849, EP-A-0 700 949, EP-A-0 761 708, JP-A-4145123, US-A-5470813, EP-A-0 743 093 und WO-A-97/40086) offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid-Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z. B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly-(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der DMC-Katalysatoren im ersten Schritt die wässrigen Lösungen des Metallsalzes (z. B. Zinkchlorid), eingesetzt im stöchiometrischen Überschuss (mindestens 50 Mol-%) bezogen auf Metallcyanidsalz, (also mindestens ein molares Verhältnis von cyanidfreiem Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält. Der organische Komplexligand kann dabei in der wässrigen Lösung des cyanidfreien Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Metallcyanidsalzes und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d. h. der Herstellung der Suspension) erfolgt unter Einsatz einer Mischdüse, besonders bevorzugt unter Einsatz eines Strahldispergators wie in WO-A-01/39883 beschrieben.

Im zweiten Schritt erfolgt die Isolierung des Feststoffs (d. h. die Vorstufe des erfindungsgemäßen Katalysators) aus der Suspension durch bekannte Techniken, wie Zentrifugation oder Filtration.

In einer bevorzugten Ausführungsvariante zur Herstellung des Katalysators wird der isolierte Feststoff anschließend in einem **dritten Verfahrensschritt** mit einer wässrigen Lösung des organischen Komplexliganden gewaschen (z. B. durch Resuspendieren und anschließende erneute Isolierung durch Filtration oder Zentrifugation). Auf diese Weise können zum Beispiel wasserlösliche Nebenprodukte, wie Kaliumchlorid, aus dem Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung weitere komplexbildende Komponente, bevorzugt im Bereich zwischen 0,5 und 5 Gew.-%, bezogen auf die Gesamtlösung, zugefügt.
Außerdem ist es vorteilhaft, den isolierten Feststoff mehr als einmal zu waschen. Hierzu kann z.B. der erste Waschvorgang wiederholt werden. Bevorzugt ist es aber, für weitere Waschvorgänge nicht wässrige Lösungen zu verwenden, z.B. eine Mischung aus organischem Komplexliganden und weiterer komplexbildender Komponente.
Der isolierte und gegebenenfalls gewaschene Feststoff wird anschließend, gegebenenfalls nach Pulverisierung, bei Temperaturen von im allgemeinen 20 - 100 °C und bei Drücken von im allgemeinen 0,1 mbar bis Normaldruck (1013 mbar) getrocknet.
Ein bevorzugtes Verfahren zur Isolierung der DMC-Katalysatoren aus der Suspension durch Filtration, Filterkuchenwäsche und Trocknung wird in WO-A-01/80994 beschrieben.

Die Konzentration an eingesetztem DMC-Katalysator beträgt 5,0 ppm bis 1000 ppm, bevorzugt 10 ppm bis 900 ppm und besonders bevorzugt 20 ppm bis 800 ppm, bezogen auf die Masse des herzustellenden Polyetherpolyols. Je nach Anforderungsprofil der nachgeschalteten Anwendung kann der DMC-Katalysator im Produkt belassen oder (teilweise) abgetrennt werden Die (teilweise) Abtrennung des DMC-Katalysators kann beispielsweise durch Behandlung mit Adsorbentien erfolgen. Verfahren zur Abtrennung von DMC-Katalysatoren sind beispielsweise beschrieben in US-A-4,987,271, DE-A-3132258, EP-A-0 406 440, US-A-5,391,722, US-A-5,099,075, US-A-4,721,818, US-A-4,877,906 und EP-A-0 385 619.

Für das erfindungsgemäße Verfahren geeignete Alkylenoxide haben 2 bis 24 Kohlenstoffatome. Bei den Alkylenoxiden mit 2 bis 24 Kohlenstoffatomen handelt es sich bevorzugt um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach Alkylenoxidierte Fette als Mono-, Di- und Triglyceride, Alkylenoxidierte Fettsäuren, $C_1$-$C_{24}$-Ester von Alkylenoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, sowie Alkylenoxidfunktionelle Alkyloxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyl-dimethoxysilan, 3-Glycidyloxypropylethyldiethoxysilan und 3-Glycidyloxypropyltriisopropoxysilan. Bevorzugt wird als Alkylenoxid wenigstens ein Akylenoxid ausgewählt aus der Gruppe bestehend aus Ethylenoxid und Propylenoxid eingesetzt.
Weitere mit Alkylenoxiden nach dem erfindungsgemäßen Verfahrens unter DMC-Katalyse co-polymerisierbare Monomere sind alle Sauerstoffhaltigen cyclischen Verbindungen, insbesondere Lactone, Lactide, aliphatische und aromatische cyclische Carbonsäureanhydride und cyclische Carbonate. Ihre Verwendung wird beschrieben in US-A-3,538,043, US-A-4,500,704, US-A-5,032,671, US-A-6,646,100, EP-A-0 222 453 und WO-A-2008/013731.
Im Folgenden werden mehrere Varianten zur Durchführung des erfindungsgemäßen Verfahrens detailliert beschrieben. Die Darstellung ist lediglich beispielhaft und nicht als die vorliegende Erfindung beschränkend zu verstehen.

In einer bevorzugten Ausführungsform der Erfindung (Variante A) werden zunächst wenigstens ein Urethan-Alkohol der Formel (II) und der Doppelmetallcyanid-Katalysator vorgelegt und anschließend das Alkylenoxid hinzugefügt.

Variante A) ("Semibatch Verfahrensweise"):

In Variante A) des erfindungsgemäßen Verfahrens wird wenigstens ein Urethan-Alkohol der Formel (II) mit dem DMC-Katalysator in einem Reaktor / Reaktorsystem zunächst vorgelegt. Dem Urethan-Alkohol können ggf. vor Inkontaktbringen mit dem DMC-Katalysator geringe Mengen einer anorganischen Mineralsäure, bevorzugt Phosphorsäure, zugesetzt werden, wie in den Anmeldeschriften WO-A-99/14258 und EP-A-1 577 334 beschrieben, um etwaige Basenspuren im Urethan-Alkohol zu neutralisieren, bzw. um den Produktionsprozess generell stabiler zu gestalten. Nach Aufheizen auf Temperaturen von 50 °C bis 160 °C, bevorzugt 60 °C bis 140 °C, ganz besonders bevorzugt 70 °C bis 140 °C wird der Reaktorinhalt in einer bevorzugten Verfahrensvariante mit Inertgas über einen Zeitraum von bevorzugt 10 bis 60 min. unter Rühren gestrippt. Beim Strippen mit Inertgas werden flüchtige Bestandteile, wie beispielsweise Wasserspuren, unter Einleiten von Inertgasen in die Flüssigphase bei gleichzeitig angelegtem Vakuum, bei einem absoluten Druck von 5 mbar bis 500 mbar, entfernt. Nach Eindosieren von typischerweise 5 Gew.-% bis 20 Gew.-% eines oder mehrerer Alkylenoxide, bezogen auf die Menge an vorgelegtem Urethan-Alkohol, wird der DMC-Katalysator aktiviert. Die Zugabe eines oder mehrerer Alkylenoxide kann vor, während oder nach dem Aufheizen des Reaktorinhaltes auf Temperaturen von 50 °C bis 160 °C, bevorzugt 60 °C bis 140 °C, ganz besonders bevorzugt 70 °C bis 140 °C geschehen; sie erfolgt bevorzugt nach dem Strippen. Die Aktivierung des Katalysators macht sich durch einen beschleunigten Abfall des Reaktordruckes bemerkbar, wodurch der beginnende Alkylenoxidumsatz angezeigt wird. Dem Reaktionsgemisch kann sodann die gewünschte Menge Alkylenoxid bzw. Alkylenoxidgemisch, kontinuierlich zugeführt

werden, wobei eine Reaktionstemperatur von 20 °C bis 200 °C, bevorzugt von 50 °C bis 160 °C, besonders bevorzugt 70 °C bis 150 °C, ganz besonders bevorzugt 80 °C bis 140 °C gewählt wird. Die Reaktionstemperatur ist in den vielen Fällen identisch mit der Aktivierungstemperatur, sie kann nach erfolgter Katalysatoraktivierung aber auch geändert werden, beispielsweise um empfindliche Starterverbindungen thermisch nicht zu stark zu belasten. Oft erfolgt die Katalysatoraktivierung bereits so schnell, dass die Dosierung einer separaten Menge Alkylenoxid zur Katalysatoraktivierung entfallen kann und direkt, gegebenenfalls zunächst mit einer reduzierten Dosierrate, mit der kontinuierlichen Dosierung eines oder mehrerer Alkylenoxide begonnen werden kann. Auch kann die Reaktionstemperatur während der gesamten Alkylenoxiddosierphase innerhalb der beschriebenen Grenzen variiert werden. Ebenfalls können die Alkylenoxide dem Reaktor auf unterschiedliche Weise zugeführt werden: Möglich ist eine Dosierung in die Gasphase oder direkt in die Flüssigphase, z.B. über ein Tauchrohr oder einen in der Nähe des Reaktorbodens in einer gut durchmischten Zone befindlichen Verteilerring. Bei DMC-katalysierten Prozessen ist die Dosierung in die Flüssigphase häufig die bevorzugte Variante. Das eine oder mehrere Alkylenoxid(e) sollten dem Reaktor kontinuierlich derart zugeführt werden, dass die sicherheitstechnischen Druckgrenzen des verwendeten Reaktorsystems nicht überschritten werden. Insbesondere bei der Codosierung von ethylenoxidhaltigen Alkylenoxidgemischen oder reinem Ethylenoxid ist darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden. Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate und Eduktvorlagen verhindert werden. Wird ein Alkylenoxidgemisch dosiert, können die jeweiligen Alkylenoxide dem Reaktor separat oder als Mischung zugeführt werden. Eine Vorvermischung der Alkylenoxide untereinander kann beispielsweise durch ein in der gemeinsamen Dosierstrecke befindliches Mischaggregat erreicht werden ("inline-blending"). Es hat sich auch bewährt, die Alkylenoxide pumpendruckseitig in einen beispielsweise über einen oder mehrere Wärmetauscher geführten Umpumpkreislauf einzeln oder vorgemischt zu dosieren. Für die gute Durchmischung mit dem Reaktionsmedium ist es dann von Vorteil ein hochscherendes Mischaggregat in den Alkylenoxid- / Reaktionsmediumstrom zu integrieren. Die Temperatur der exothermen ringöffnenden Additionsreaktion wird durch Kühlung auf dem gewünschten Niveau gehalten. Gemäß dem Stand der Technik zur Auslegung von Polymerisationsreaktoren für exotherme Reaktionen (z. B. Ullmann's Encyclopedia of Industrial Chemistry, Vol. B4, pp 167ff, 5th Ed., 1992) erfolgt eine solche Kühlung im Allgemeinen über die Reaktorwand (z. B. Doppelmantel, Halbrohrschlange) sowie mittels weiterer intern im Reaktor und/oder extern im Umpumpkreislauf angeordneter Wärmetauscherflächen, z. B. an Kühlschlangen, Kühlkerzen, Platten- Rohrbündel- oder Mischerwärmetauschern. Diese sollten so ausgelegt sein, dass auch zu Beginn der Dosierphase, d. h. bei kleinem Füllstand, effektiv gekühlt werden kann.

Generell sollte in allen Reaktionsphasen durch Auslegung und Einsatz handelsüblicher Rührorgane für eine gute Durchmischung des Reaktorinhaltes gesorgt werden, wobei hier insbesondere ein- oder mehrstufig angeordnete Rührer oder großflächig über die Füllhöhe wirkende Rührertypen, wie beispielsweise Gitterrührer, geeignet sind (siehe z. B. Handbuch Apparate; Vulkan-Verlag Essen, 1. Aufl. (1990), S. 188 - 208). Technisch besonders relevant ist hierbei eine im Mittel über den gesamten Reaktorinhalt eingetragene spezifische Mischleistung, die im Allgemeinen im Bereich von 0,2 W/l bis 5 W/l, bezogen auf das Reaktorvolumen, liegt, mit entsprechend höheren lokalen Leistungseinträgen im Bereich der Rührorgane selbst und ggf. bei niedrigeren Füllständen. Um eine optimale Rührwirkung zu erzielen, können im Reaktor gemäß allgemeinem Stand der Technik Kombinationen aus Stromstörern (z. B. Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Bevorzugt werden Rührorgane mit bodengängigen Rührstufen eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restalkylenoxide zusätzlich im Vakuum bei absoluten Drücken im Bereich von 1 mbar bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und gegebenenfalls weitere Stützlager im Behälter auf. Der Antrieb der Rührerwelle kann dabei von oben oder unten erfolgen (mit zentrischer oder exzentrischer Anordnung der Welle).

Alternativ ist es auch möglich, die notwendige Durchmischung ausschließlich mittels eines über einen Wärmetauscher geführten Umpumpkreislaufs zu erzielen oder diesen zusätzlich zum Rühraggregat als weitere Mischkomponente zu betreiben, wobei der Reaktorinhalt nach Bedarf (typischerweise 1 bis 50 mal pro Stunde) umgepumpt wird. Die mittels Umpumpung, beispielsweise über einen außenliegenden Wärmetauscher über diesen oder bei Rückführung in den Reaktor über eine Düse oder Injektor eingetragene spezifische Mischenergie beläuft sich ebenfalls auf Werte von im Mittel 0,2 bis 5 W/l, wobei diese auf das im Reaktor und den Umpumpkreislauf am Ende der Reaktionsphase befindliche Flüssigkeitsvolumen bezogen ist.

Für die Durchführung des erfindungsgemäßen Verfahrens sind die unterschiedlichsten Reaktortypen geeignet. Vorzugsweise werden zylinderförmige Behälter eingesetzt, welche ein Höhen-/Durchmesserverhältnis von 1,0 : 1 bis 10:1 besitzen. Als Reaktorböden kommen beispielsweise Kugel-, Klöpper-, Flach,- oder Konusböden in Frage.
Nach Ende der Dosierung des einen oder mehrerer Alkylenoxide kann sich eine Nachreaktionsphase anschließen, in der restliches Alkylenoxid abreagiert. Das Ende dieser Nachreaktionsphase ist erreicht, wenn kein weiterer Druckabfall im Reaktionskessel feststellbar ist. Spuren unreagierter Alkylenoxide können nach der Reaktionsphase gegebenenfalls im Vakuum bei einem absoluten Druck von 1 mbar bis 500 mbar oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum (beispielsweise durch Durchleiten von Inertgas bei einem Absolutdruck von 5 mbar bis 500 mbar) entfernt. Das Entfernen flüchtiger Bestandteile, wie beispielsweise nicht umgesetzter Alkylenoxide, entweder im Vakuum oder durch Strippen, erfolgt bei Temperaturen von 20 °C bis 200 °C, bevorzugt bei 50 °C bis 160 °C und vorzugsweise unter Rühren. Solche Strippvorgänge können auch in sog. Strippkolonnen durchgeführt werden, in denen dem Produktstrom ein Inertgas- oder Wasserdampfstrom entgegengeleitet wird. Bevorzugt wird das Strippen mit Inertgasen in Abwesenheit von Wasserdampf durchgeführt.
Nach Erreichen von Druckkonstanz bzw. nach Entfernen flüchtiger Bestandteile durch Vakuum und/oder Strippen kann das nach dem erfindungsgemäßen Verfahren erhaltene Produkt aus dem Reaktor abgelassen werden.
Ein Charakteristikum von DMC-Katalysatoren ist ihre ausgeprägte Empfindlichkeit gegen hohe Konzentrationen an Hydroxylgruppen, welche in gängigen großtechnischen Verfahren zur Polyetherpolyolherstellung beispielsweise durch zu Reaktionsbeginn im Reaktionsgemisch vorliegende hohe Anteile an Startern wie Ethylenglykol, Propylenglykol, Glycerin, Trimethylolpropan, Sorbitol oder Saccharose hervorgerufen werden, und polare Verunreinigungen des Reaktionsgemisches bzw. des Starters oder der Starter. Die DMC-Katalysatoren können dann während der Reaktionsinitiierungsphase nicht in die polymerisationsaktive Form überführt werden. Verunreinigungen können beispielsweise Wasser, Verbindungen mit einer hohen Zahl in enger Nachbarschaft stehender Hydroxylgruppen wie Kohlenhydrate und Kohlenhydratderivate oder Verbindungen mit basischen Gruppen wie beispielsweise Amine sein. Für das erfindungsgemäße Verfahren ist von besonderer Bedeutung, dass auch Substanzen mit zu Hydroxylgruppen benachbarten Urethangruppen sich nachteilig auf die Katalysatoraktivität auswirken. Um Starter mit hohen Konzentrationen an OH-Gruppen, bzw. Starter mit als Katalysatorgiften anzusehenden Verunreinigungen oder Starter mit die Katalysatoraktivität unvorteilhaft beeinflussenden Konstellationen funktioneller Gruppen dennoch DMC-katalysierten Alkylenoxidadditionsreaktionen unterziehen zu können, müssen die Hydroxylgruppenkonzentration gesenkt, die Starterkonzentration reduziert, bzw. die Katalysatorgifte unschädlich gemacht werden. Hierzu können beispielsweise aus diesen Starterverbindungen mittels basischer Katalyse zunächst Vorpolymerisate hergestellt werden, welche dann nach Aufarbeitung mittels DMC-Katalyse in die erwünschten Alkylenoxidadditionsprodukte hoher Molmasse überführt werden. Nachteilig bei dieser Vorgehensweise ist, dass solche oft mittels basischer Katalyse erhaltenen Vorpolymerisate sehr sorgfältig aufgearbeitet werden müssen, um die Deaktivierung des DMC-Katalysators durch über die Vorpolymerisate eingeschleppte basische Katalysatorspuren auszuschließen.

Diese Nachteile können durch das Verfahren der kontinuierlichen Starterzudosierung, welches in WO-A-97/29146 offenbart ist, überwunden werden. Hierbei werden kritische Verbindungen im Reaktor nicht vorgelegt, sondern neben den Alkylenoxiden dem Reaktor während der Reaktion kontinuierlich zugeführt. Als Startmedium, bzw. als sogenannte H-funktionelle Starterpolyole **S-I** für die Reaktion können in diesem Verfahren Alkylenoxidadditionsprodukte H-funktioneller Starterverbindungen, beispielsweise auch solcher ohne Urethangruppen, vorgelegt werden. Es ist auch die Verwendung des nach dem erfindungsgemäßen Verfahren hergestellten Polyetherpolyols selbst, das vorher separat hergestellt wurde, als Startmedium (S-I) möglich. Die Notwendigkeit, für weitere Alkylenoxidadditionen geeignete Vorpolymerisate zunächst separat herstellen zu müssen, entfällt somit.

<u>Variante B) ("CAOS-Semibatch Verfahrensweise"):</u>

In Variante B) des erfindungsgemäßen Verfahrens werden ein H-funktionelles Starterpolyol **S-I** und der DMC-Katalysator im Reaktorsystem vorgelegt, und wenigstens ein Urethan-Alkohol der Formel (II) wird kontinuierlich gemeinsam mit einem oder mehreren Alkylenoxiden zugeführt. Als H-funktionelles Starterpolyol **S-I** für diese Variante sind Alkylenoxidadditionsprodukte wie beispielsweise Polyetherpolyole, Polycarbonatpolyole, Polyestercarbonatpolyole oder Polyethercarbonatpolyole, jeweils beispielsweise mit OH-Zahlen im Bereich von 3,0 mg KOH/g bis 1000 mg KOH/g, vorzugsweise von 3,0 mg KOH/g bis 300 mg KOH/g, und / oder ein gemäß dem erfindungsgemäßen Verfahren separat hergestelltes Polyetherpolyol, geeignet. Vorzugsweise wird ein gemäß dem erfindungsgemäßen Verfahren separat hergestelltes Polyetherpolyol als H-funktionelles Starterpolyol **S-I** eingesetzt.
Vorzugsweise wird die Dosierung des wenigstens einen Urethan-Alkohols und die eines oder mehrerer Alkylenoxides / Alkylenoxide gleichzeitig beendet, oder der Urethan-Alkohol und eine erste Teilmenge an einem oder mehreren Alkylenoxid(en) werden zunächst gemeinsam zudosiert und anschließend die zweite Teilmenge an einem oder mehreren Alkylenoxiden, wobei die Summe der ersten und zweiten Teilmenge an einem oder mehreren Alkylenoxiden der Gesamtmenge der eingesetzten ein oder mehreren Alkylenoxiden entspricht. Die erste Teilmenge beträgt vorzugsweise

60 Gew.-% bis 98 Gew. -% und die zweite Teilmenge beträgt 40 Gew.-% bis 2 Gew.-% der insgesamt zu dosierenden Menge eines oder mehrerer Alkylenoxide. Wird die Zusammensetzung des Alkylenoxiddosierstroms nach Ende der Dosierung des Urethan-Alkohols geändert, lassen sich auch nach Verfahrensvariante B) Produkte mit Multiblockstrukturen herstellen. Nach Zudosierung der Reagenzien kann sich eine Nachreaktionsphase anschließen, in der der Verbrauch an Alkylenoxid durch Überwachung des Drucks quantifiziert werden kann. Nach Erreichen von Druckkonstanz kann das Produkt, gegebenenfalls nach Anlegen von Vakuum oder durch Strippen zur Entfernung von nicht umgesetzten Alkylenoxiden, wie oben beschrieben, abgelassen werden.
Es ist alternativ gemäß Variante B des erfindungsgemäßen Verfahrens auch möglich, zusätzlich zum Urethan-Alkohol auch die oben beschriebenen H-funktionellen Starterverbindungen, die keine Urethan-Alkohole sind, kontinuierlich gemeinsam mit einem oder mehreren Alkylenoxiden einzusetzen.

<u>Variante C ("CAOS-Conti Verfahrensweise"):</u>

In einer weiteren bevorzugten Ausführungsform des erfindungsgemäßen Verfahrens (Variante C) wird ein H-funktionelles Starterpolyol **S-I** und eine Teilmenge des Doppelmetallcyanid-Katalysators vorgelegt, und dann wenigstens ein Urethan-Alkohol der Formel (II), sowie weiterer Doppelmetallcyanid-Katalysator gemeinsam mit dem Alkylenoxid kontinuierlich zugeführt, wobei das hierbei entstandene Polyetherpolyol kontinuierlich dem Reaktorsystem nach einer vorwählbaren mittleren Verweilzeit ent¬nommen wird.

In Variante C) des erfindungsgemäßen Verfahrens werden die Polyetherpolyole vollkontinuierlich hergestellt. Ein vollkontinuierliches Verfahren zur Herstellung von Alkylenoxid-Additionsprodukten ist in WO-A-98/03571 grundsätzlich beschrieben. Die dort offenbarte Verfahrensweise ist für die Durchführung des erfindungsgemäßen Verfahrens anwendbar. In dieser Variante werden neben einem oder mehreren Alkylenoxiden und wenigstens einem Urethan-Alkohol auch der DMC-Katalysator dem Reaktor bzw. einem Reaktorsystem unter Alkoxylierungsbedingungen kontinuierlich zugeführt und das Polyetherpolyol kontinuierlich dem Reaktor bzw. dem Reaktorsystem nach einer vorwählbaren mittleren Verweilzeit entnommen. Zum Anfahren eines solchen vollkontinuierlichen Prozesses wird ein Starterpolyol **S-I** und eine Teilmenge des DMC-Katalysators vorgelegt. Als Starterpolyol **S-I** für Variante C) des erfindungsgemäßen Verfahrens sind Alkylenoxidadditionsprodukte wie beispielsweise Polyetherpolyole, Polycarbonatpolyole, Polyestercarbonatpolyole, Polyethercarbonatpolyole beispielsweise mit OH-Zahlen im Bereich von 3,0 mg KOH/g bis 1000 mg KOH/g, vorzugsweise von 3,0 mg KOH/g bis 300 mg KOH/g, und/oder nach dem erfindungsgemäßen Verfahren hergestelltes Polyetherpolyol, das vorher separat hergestellt wurde, geeignet. Vorzugsweise wird nach dem erfindungsgemäßen Verfahren hergestelltes Polyetherpolyol, das vorher separat hergestellt wurde, als Starterpolyol in Variante C des erfindungsgemäßen Verfahrens eingesetzt.
Beispielsweise wird der Reaktor so betrieben, dass dieser vollständig mit dem Reaktionsgemisch gefüllt ist ("Liquid Full"-Fahrweise).
Es können sich kontinuierliche Nachreaktionsschritte, beispielsweise in einer Reaktorkaskade oder in einem Rohrreaktor anschließen. Flüchtige Bestandteile können im Vakuum und/oder durch Strippen, wie oben beschrieben, entfernt werden.
Beispielsweise kann in einem nachfolgenden Schritt das kontinuierlich entfernte Reaktionsgemisch, welches im allgemeinen einen Gehalt von 0,05 Gew.-% bis 10 Gew.-% Alkylenoxid enthält, in einen Nachreaktor überführt werden, in dem im Wege einer Nachreaktion der Gehalt an freiem Alkylenoxid auf weniger als 0,05 Gew.-% im Reaktionsgemisch reduziert wird. Als Nachreaktor kann beispielsweise ein Rohrreaktor, ein Schlaufenreaktor oder ein Rührkessel dienen. Bevorzugt liegt der Druck in diesem Nachreaktor bei demselben Druck wie in dem Reaktionsapparat, in dem der vorhergehende Reaktionsschritt der Anlagerung der Alkylenoxide an Urethan-Alkohol durchgeführt wird. Die Temperatur in dem nachgeschalteten Reaktor liegt bevorzugt bei 50 bis 150°C und besonders bevorzugt bei 80 bis 140°C.

In besonders bevorzugten Ausführungsformen der Varianten B und C des erfindungsgemäßen Verfahrens wird als Starterpolyol S-I ein erfindungsgemäßes Polyetherpolyol oder ein Polyetherpolyol erhältlich nach dem erfindungsgemäßen Verfahren eingesetzt.

Ein weiterer Gegenstand der vorliegenden Erfindung ist ein Polyetherpolyol erhältlich nach dem erfindungsgemäßen Verfahren.
Die OH-Zahlen der erhaltenen Polyetherpolyole weisen bevorzugt Werte von 3 mg KOH/g bis 400 mg KOH/g, besonders bevorzugt von 10 mg KOH/g bis 200 mg KOH/g, ganz besonders bevorzugt von 20 mg KOH/g bis 150 mg KOH/g, auf. Dies gilt unabhängig von der eingesetzten Verfahrensvariante (A, B oder C).

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien steht sie in folgender Beziehung zur OH-Zahl:

$$\text{Äquivalentmolmasse} = 56100 / \text{OH-Zahl [mg KOH/g]}$$

Den nach dem erfindungsgemäßen Verfahren erhältlichen Polyetherpolyolen können gegebenenfalls Alterungsschutzmittel wie z. B. Antioxidantien zugesetzt werden.

Die vorliegende Erfindung betrifft ferner die Verwendung eines erfindungsgemäßen Polyetherpolyols zur Herstellung eines Polyurethanpolymers, bevorzugt eines Polyurethan-Weichschaums, besonders bevorzugt eines Polyurethan-Weichblockschaumstoffs oder eines Polyurethan-Weichformschaumstoffs.

Ein weiterer Gegenstand der Erfindung ist ein Polyurethanpolymer, bevorzugt ein Polyurethan-Weichschaum, besonders bevorzugt ein Polyurethan-Weichblockschaumstoff oder ein Polyurethan-Weichformschaumstoff, erhältlich durch Umsetzung eines Polyisocyanats mit einem erfindungsgemäßen Polyetherpolyol nach einem dem Fachmann geläufigen Verfahren unter Zuhilfenahme üblicher Additive wie beispielsweise Aktivatoren, Stabilisatoren, Treibmittel, Vernetzer, Kettenverlängerer und/oder Füllstoffen sowie ggf. weiterer Polyetherpolyole, Polyesterpolyole, Polyethercarbonatpolyole, Polycarbonatpolyole und/oder füllstoffhaltiger Polyole (Polymerpolyole, Polyharnstoffdispersionen etc.). Geeignete Polyisocyanate sind aliphatische, cycloaliphatische, araliphatische, aromatische und heterocyclische Polyisocyanate, wie sie z.B. von W. Siefken in Justus Liebigs Annalen der Chemie, 562, Seiten 75 bis 136 beschrieben werden, beispielsweise solche der Formel (XI),

$$Q(NCO)_n, \qquad (XI)$$

in der

> n = 2 - 4, vorzugsweise 2 -3,
> und
> Q einen aliphatischen Kohlenwasserstoffrest mit 2 - 18, vorzugsweise 6 - 10 C-Atomen, einen cycloaliphatischen Kohlenwasserstoffrest mit 4 - 15, vorzugsweise 6 - 13 C-Atomen oder einen araliphatischen Kohlenwasserstoffrest mit 8 - 15, vorzugsweise 8 - 13 C-Atomen bedeuten.

Beispielsweise handelt es sich um solche Polyisocyanate, wie sie in der EP 0 007 502 A1, Seiten 7 - 8, beschrieben werden. Bevorzugt werden in der Regel die technisch leicht zugänglichen Polyisocyanate, z.B. das 2,4- und 2,6-Toluylendiisocyanat, sowie beliebige Gemische dieser Isomeren ("TDI"); Polyphenylpolymethylenpolyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten. Die urethangruppenhaltigen Polyisocyanate (Präpolymere) können beispielsweise Reaktionsprodukte der Polyisocyanate mit Polyester-Polyolen oder aber beliebigen anderen Polyolen (beispielsweise konventionellen Polyetherpolyolen) sein. Vorzugsweise wird als Polyisocyanat mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt, besonders bevorzugt wird als Polyisocyanat eine Mischung enthaltend 4,4'-Diphenylmethandiisocyanat und 2,4'-Diphenylmethandiisocyanat und Polyphenylpolymethylenpolyisocyanat eingesetzt.

Neben den vorgenannten Polyisocyanaten können für die Herstellung der Polyurethanpolymere zusätzlich noch konventionelle Polyetherpolyole verwendet werden. Unter konventionellen Polyetherpolyolen im Sinne der Erfindung sind Verbindungen zu verstehen, die Alkylenoxidadditionsprodukte von Starterverbindungen mit Zerewitinoff-aktiven Wasserstoffatomen sind. Beispiele für solche Polyetherpolyole sind dem Fachmann bekannt. Sie können eine Hydroxylzahl gemäß DIN 53240 von $\geq$ 3,0 mg KOH/g bis $\leq$ 1000 mg KOH/g, vorzugsweise von $\geq$ 5,0 mg KOH/g bis $\leq$ 600 mg KOH/g aufweisen. Die für die Herstellung der konventionellen Polyetherpolyole eingesetzten Starterverbindungen mit Zerewitinoff-aktiven Wasserstoffatomen weisen meist Funktionalitäten von 2 bis 8 auf. Die Starterverbindungen können hydroxyfunktionell und / oder aminofunktionell sein. Beispiele für hydroxyfunktionelle Starterverbindungen sind Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Triethanolamin, Pentaerythrit, Sorbitol, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol oder Melamin oder Harnstoff. Beispiele für aminofunktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans und bei der Kondensation von Anilin mit Formaldehyd zu Diaminodiphenylmethan anfallende höherkernige Produkte.

Geeignete Alkylenoxide für die konventionellen Polyetherpolyole sind beispielsweise Ethylenoxid, Propylenoxid, 1,2-Butylenoxid bzw. 2,3-Butylenoxid und Styroloxid. Bevorzugt werden Propylenoxid und Ethylenoxid dem Reaktionsgemisch einzeln, im Gemisch oder nacheinander zugeführt. Werden die Alkylenoxide nacheinander dosiert, so

enthalten die hergestellten Produkte Polyetherketten mit Blockstrukturen. Produkte mit Ethylenoxidendblöcken sind beispielsweise durch erhöhte Konzentrationen an primären Endgruppen gekennzeichnet, welche den Systemen eine vorteilhafte Isocyanatreaktivität verleihen.

Die Herstellung der konventionellen Polyetherpolyole kann basenkatalysiert, beispielsweise über Alkalimetallhydroxid- oder Aminkatalyse, doppelmetallcyanidkatalysiert oder Lewis- bzw. Brønsted-säurekatalysiert erfolgen.

Neben den vorgenannten konventionellen Polyetherpolyolen können für die Herstellung der Polyurethanpolymere zusätzlich oder alternativ auch Polyesterpolyole verwendet werden. Geeignete Polyesterpolyole weisen vorzugsweise OH-Zahlen im Bereich von 6 bis 800 mg KOH/g auf und können beispielsweise aus mehrfunktionellen Carbonsäuren, vorzugsweise organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden. Anstelle der mehrfunktionellen Carbonsäuren können auch deren Derivate, wie z. B. Säurechloride oder - anhydride eingesetzt werden.

## Beispiele:

## Messmethoden:

Experimentell ermittelte OH-Zahlen wurden gemäß der Vorschrift der DIN 53240 bestimmt. Die Aminzahlen (NH-Zahl) wurden gemäß der Vorschrift der DIN 53176 ermittelt.

Die Viskositäten wurden mittels Rotationsviskosimeter (Physica MCR 51, Hersteller: Anton Paar) nach der Vorschrift der DIN 53018 ermittelt.

Die Bestimmung der Funktionalität des Starters im fertigen Polyetherpolyol wurde mittels $^{13}$C-NMR (Firma Bruker, Advance 400, 400 MHz; Wartezeit d1: 4s, 6000 Scans) durchgeführt. Die Probe wurde jeweils in deuteriertem Aceton-D6 unter Zusatz von Crom(III)acetylacetonat gelöst. Die Lösungskonzentration lag bei 500 mg / mL.

Die relevanten Resonanzen im $^{13}$C-NMR (bezogen auf $CHCl_3$ = 7,24 ppm) sind wie folgt: Ausgewertet werden die Kohlenstoffsignale der unmittelbar am Stickstoff gebundenen C-Atome (Methylengruppen, Methingruppe) des Starters:

| | |
|---|---|
| Bifunktionell gestartet: | 40,4 ppm bis 40,0 ppm (ein Kohlenstoff) |
| Trifunktionell gestartet: | 42,2 ppm bis 40,5 ppm (zwei Kohlenstoffe) |

Bifunktionell gestartet bedeutet, dass nur die OH-Gruppen der Urethan-Alkohol Starterverbindung alkoxyliert werden.

Trifunktionell gestartet bedeutet, dass die OH-Gruppen sowie die NH-Gruppe der Urethanbindung der Urethan-Alkohol Starterverbindung alkoxyliert werden.

Die chemische Verschiebungen im $^{13}$C-NMR wurden mit Vergleichsmessungen (Vergleichsspektren) ermittelt.

Die Rohdichten wurde gemäß DIN EN ISO 845 ermittelt.

Die Stauchhärten (40% Kompression) gemäß DIN EN ISO 1798 ermittelt.

## Eingesetzte Rohstoffe:

### Katalysator für die Alkylenoxidaddition (DMC-Katalysator):

Doppelmetallcyanid-Katalysator, enthaltend Zinkhexacyanocobaltat, tert.-Butanol und Polypropylenglykol mit einem zahlenmittleren Molekulargewicht von 1000 g/mol; gemäß Beispiel 6 in WO-A 01/80994.

Cyclisches Propylencarbonat (cPC): Fa. Acros, Art.Nr.: 131560025

Cyclisches Ethylencarbonat (cEC): Fa. Acros, Art.Nr.: 118410010 Ethanolamin: Fa. Merck; Art-Nr: 800849

Stabilisator 1: Siloxan-basierter Schaumstabilisator Tegostab® BF 2370, Evonik Goldschmidt

Isocyanat 1: Gemisch aus 80 Gewichts-% 2,4- und 20 Gewichts-% 2,6-Toluylendiisocyanat, erhältlich unter dem Namen Desmodur® T 80, Bayer MaterialScience AG

Katalysator 1: Bis-(2-dimethylamino-ethyl)-ether in Dipropylenglykol, erhältlich als Addocat® 108, Fa. Rheinchemie

Katalysator 2: Zinn(II)-ethylhexanoat, erhältlich als Dabco® T-9, Fa. Air Products

**Herstellung von Urethan-Alkoholen:**

**Beispiel 1a:**

In einem 10-L-Vierhalskolben mit Rückflusskühler und Thermometer wurde cyclisches Propylencarbonat (6080 g, 59,6 mol) vorgelegt. Anschließend wurde bei 60°C Ethanolamin (2405 g, 39,6 mol) langsam innerhalb 50 min so zu getropft, dass die Temperatur 72°C nicht überschritt. Die Reaktion wurde anschließend für insgesamt 24 h bei 60°C nachgerührt. Nach Abkühlen auf 25°C wurde der Urethan-Alkohol erhalten.

| Produkteigenschaften des resultierenden Urethan-Alkohols: | |
|---|---|
| OH-Zahl: | 507 mg KOH/g |
| NH-Zahl: | 0,51 mg KOH/g |
| Viskosität (25°C): | 268 mPas |

**Beispiel 1b:**

Ein Teil des Produktes wurde mittels Dünnschichtverdampfung (0,1 mbar, 120 °C) von flüchtigen Bestandteilen befreit.

| Produkteigenschaften: | |
|---|---|
| OH-Zahl: | 671 mg KOH/g |
| NH-Zahl: | 0,20 mg KOH/g |
| Viskosität (25°C): | 3170 mPas |

**Beispiel 2:**

In einem 2-L-Vierhalskolben mit Rückflusskühler und Thermometer wurde ein Gemisch aus cyclischem Propylencarbonat (1181 g, 11,6 mol) und auf 50°C erwärmtem cyclischem Ethylencarbonat (62 g, 0,7 mol) vorgelegt. Anschließend wurde bei 60°C Ethanolamin (500 g, 8,2 mol) langsam innerhalb 60 min so zu getropft, dass die Temperatur 70°C nicht überschritt. Die Reaktion wurde anschließend für insgesamt 15 h bei 60°C nachgerührt. Nach Abkühlen auf 25°C wurde der Urethan-Alkohol erhalten

| Produkteigenschaften: | |
|---|---|
| OH-Zahl: | 523 mg KOH/g |
| NH-Zahl: | 0,20 mg KOH/g |
| Viskosität (25°C): | 313 mPas |

**Beispiel 3:**

In einem 2-L-Vierhalskolben mit Rückflusskühler und Thermometer wurde ein Gemisch aus cyclischem Propylencarbonat (1110 g, 10,9 mol) und auf 50 °C erwärmtem cyclischem Ethylencarbonat (123 g, 1,4 mol) vorgelegt. Anschließend wurde bei 60°C Ethanolamin (500 g, 8,2 mol) langsam innerhalb 60 min so zu getropft, dass die Temperatur 79°C nicht überschritt. Die Reaktion wurde anschließend für insgesamt 15 h bei 60°C nachgerührt. Nach Abkühlen auf 25°C wurde der Urethan-Alkohol erhalten

| Produkteigenschaften: | |
|---|---|
| OH-Zahl: | 527 mg KOH/g |
| NH-Zahl: | 0,30 mg KOH/g |
| Viskosität (25°C): | 295 mPas |

**Herstellung von Polyetherpolyolen:**

**Beispiel 4 (CAOS-Semibatch-Verfahren):**

In einem 2 Liter-Edelstahldruckreaktor wurden 200 g Polypropylenglykol mit Molmasse = 2000 g/mol und 36 mg DMC-Katalysator unter Stickstoff vorgelegt und auf 130°C temperiert. Zum Strippen wurde in das Reaktionsgemisch bei 130°C für eine Dauer von 30 min Stickstoff eingeleitet und gleichzeitig ein reduzierter Druck (absolut) angelegt, so dass sich im Reaktor ein reduzierter Druck von 0,1 bar (absolut) einstellte. Dann wurden bei 130°C unter Rühren (800 U/min) zunächst 20 g Propylenoxid innerhalb von 5 min in den Reaktor dosiert. Anschließend wurden über einen Zeitraum von 6,5 h 838 g Propylenoxid und 122 g Urethan-Alkohol aus Beispiel 1a bei 130°C unter Rühren (800 U/min) in den Reaktor dosiert. Abschließend wurden bei 130°C unter Rühren (800 U/min) innerhalb von 30 min noch weitere 20 g Propylenoxid in den Reaktor dosiert. Nach einer Nachreaktionszeit von 30 min bei 130°C wurden leicht flüchtige Anteile bei 130°C für 60 min unter vermindertem Druck bei 50 mbar (absolut) abdestilliert und das Reaktionsgemisch anschließend auf Raumtemperatur abgekühlt.

| Produkteigenschaften: | |
|---|---|
| OH-Zahl: | 53,3 mg KOH/g |
| Viskosität (25°C): | 749 mPas |

**Beispiel 5 (CAOS-Semibatch-Verfahren):**

In einem 2 Liter-Edelstahldruckreaktor wurden 200 g Polypropylenglykol mit Molmasse = 2000 g/mol und 36 mg DMC-Katalysator unter Stickstoff vorgelegt und auf 130°C temperiert. Zum Strippen wurde in das Reaktionsgemisch bei 130°C für eine Dauer von 30 min Stickstoff eingeleitet und gleichzeitig ein reduzierter Druck (absolut) angelegt, so dass sich im Reaktor ein reduzierter Druck von 0,1 bar (absolut) einstellte. Dann wurden bei 130°C unter Rühren (800 U/min) zunächst 20 g Propylenoxid innerhalb von 5 min in den Reaktor dosiert. Anschließend wurden über einen Zeitraum von 7 h 876 g Propylenoxid und 84 g Urethan-Alkohol aus Beispiel 1b bei 130°C unter Rühren (800 U/min) in den Reaktor dosiert. Abschließend wurden bei 130°C unter Rühren (800 U/min) innerhalb von 10 min noch weitere 20 g Propylenoxid in den Reaktor dosiert. Nach einer Nachreaktionszeit von 30 min bei 130°C wurden leicht flüchtige Anteile bei 130°C für 60 min unter vermindertem Druck bei 50 mbar (absolut) abdestilliert und das Reaktionsgemisch anschließend auf Raumtemperatur abgekühlt.

| Produkteigenschaften: | |
|---|---|
| OH-Zahl: | 44,5 mg KOH/g |
| Viskosität (25°C): | 764 mPas |

**Beispiel 6 (CAOS-Semibatch-Verfahren):**

In einem 2 Liter-Edelstahldruckreaktor wurden 200 g Polypropylenglykol mit Molmasse = 2000 g/mol und 36 mg DMC-Katalysator unter Stickstoff vorgelegt und auf 130°C temperiert. Zum Strippen wurde in das Reaktionsgemisch bei 130°C für eine Dauer von 30 min Stickstoff eingeleitet und gleichzeitig ein reduzierter Druck (absolut) angelegt, so dass sich im Reaktor ein reduzierter Druck von 0,1 bar (absolut) einstellte. Dann wurden bei 130°C unter Rühren (800 U/min) zunächst 20 g Propylenoxid innerhalb von 5 min in den Reaktor dosiert. Anschließend wurden über einen Zeitraum von 6,5 h 838 g Propylenoxid und 122 g Urethan-Alkohol aus Beispiel 2 bei 130°C unter Rühren (800 U/min) in den Reaktor dosiert. Abschließend wurden bei 130°C unter Rühren (800 U/min) innerhalb von 10 min noch weitere 20 g Propylenoxid in den Reaktor dosiert. Nach einer Nachreaktionszeit von 30 min bei 130°C wurden leicht flüchtige Anteile bei 130°C für 60 min unter vermindertem Druck bei 50 mbar (absolut) abdestilliert und das Reaktionsgemisch anschließend auf Raumtemperatur abgekühlt.

| Produkteigenschaften: | |
|---|---|
| OH-Zahl: | 49,3 mg KOH/g |
| Viskosität (25°C): | 641 mPas |

**Beispiel 7 (CAOS-Semibatch-Verfahren):**

In einem 2 Liter-Edelstahldruckreaktor wurden 200 g Polypropylenglykol mit Molmasse = 2000 g/mol und 36

mg DMC-Katalysator unter Stickstoff vorgelegt und auf 130°C temperiert. Zum Strippen wurde in das Reaktionsgemisch bei 130°C für eine Dauer von 30 min Stickstoff eingeleitet und gleichzeitig ein reduzierter Druck (absolut) angelegt, so dass sich im Reaktor ein reduzierter Druck von 0,1 bar (absolut) einstellte. Dann wurden bei 130°C unter Rühren (800 U/min) zunächst 20 g Propylenoxid innerhalb von 5 min in den Reaktor dosiert. Anschließend wurden über einen Zeitraum von 7 h 838 g Propylenoxid und 122 g Urethan-Alkohol aus Beispiel 3 bei 130°C unter Rühren (800 U/min) in den Reaktor dosiert. Abschließend wurden bei 130°C unter Rühren (800 U/min) innerhalb von 10 min noch weitere 20 g Propylenoxid in den Reaktor dosiert. Nach einer Nachreaktionszeit von 30 min bei 130°C wurden leicht flüchtige Anteile bei 130°C für 60 min unter vermindertem Druck bei 50 mbar (absolut) abdestilliert und das Reaktionsgemisch anschließend auf Raumtemperatur abgekühlt.

| Produkteigenschaften: | |
|---|---|
| OH-Zahl: | 49,9 mg KOH/g |
| Viskosität (25°C): | 677 mPas |

**Beispiel 8 (CAOS-Semibatch-Verfahren, "P2P"):**

In einem 2 Liter-Edelstahldruckreaktor wurden 200 g des Polyetherpolyols aus Beispiel 6 und 30 mg DMC-Katalysator unter Stickstoff vorgelegt und auf 130°C temperiert. Zum Strippen wurde in das Reaktionsgemisch bei 130°C für eine Dauer von 30 min Stickstoff eingeleitet und gleichzeitig ein reduzierter Druck (absolut) angelegt, so dass sich im Reaktor ein reduzierter Druck von 0,1 bar (absolut) einstellte. Dann wurden bei 130°C unter Rühren (800 U/min) zunächst 20 g Propylenoxid innerhalb von 5 min in den Reaktor dosiert. Anschließend wurden über einen Zeitraum von 7 h 838 g Propylenoxid und 122 g Urethan-Alkohol aus Beispiel 2 bei 130°C unter Rühren (800 U/min) in den Reaktor dosiert. Abschließend wurden bei 130°C unter Rühren (800 U/min) innerhalb von 10 min noch weitere 20 g Propylenoxid in den Reaktor dosiert. Nach einer Nachreaktionszeit von 30 min bei 130°C wurden leicht flüchtige Anteile bei 130°C für 60 min unter vermindertem Druck bei 50 mbar (absolut) abdestilliert und das Reaktionsgemisch anschließend auf Raumtemperatur abgekühlt.

| Produkteigenschaften: | |
|---|---|
| OH-Zahl: | 53,6 mg KOH/g |
| Viskosität (25°C): | 635 mPas |

**Beispiel 9 (CAOS-Conti-Verfahren):**

In einen kontinuierlichen, in "Liquid-Full" Fahrweise (d.h. der Reaktor ist vollständig mit dem Reaktionsgemisch gefüllt) betriebenen 2 Liter-Edelstahldruckreaktor, in dem 2000 g eines trifunktionellen Poly(oxypropylen)polyols mit Molmasse 3500 g/mol enthaltend 25 ppm aktivierten DMC-Katalysator vorgelegt worden waren, wurden bei 130°C unter Rühren (800 U/min) über einen Zeitraum von 24 h folgende Komponenten mit den angegebenen Dosierraten dosiert:

- Propylenoxid mit 797 g/h
- Ethylenoxid mit 97 g/h
- Mischung aus 58 g Urethan-Alkohol aus Beispiel 1a und 48 mg DMC-Katalysator mit 58 g/h.

Die Reaktionsmischung wurde über eine Produktaustragsleitung kontinuierlich aus dem Reaktor entnommen und zur Vervollständigung der Reaktion in einen auf 130°C temperierten 1 Liter-Rohrnachreaktor überführt. Aus dem erhaltenen Reaktionsgemisch wurden leicht flüchtige Anteile bei 130°C für 60 min unter vermindertem Druck bei 50 mbar (absolut) abdestilliert und anschließend auf Raumtemperatur abgekühlt.
Eine nach 24 h Reaktionszeit gezogene Probe wurde analysiert.

| Produkteigenschaften: | |
|---|---|
| OH-Zahl: | 31,4 mg KOH/g |
| Viskosität (25°C): | 1302 mPas |

OH-Funktionalität: ca. 2,9 (bestimmt mittels $^{13}C$-NMR)

**Beispiel 10 (CAOS-Conti-Verfahren:)**

In einen kontinuierlichen, in "Liquid-Full" Fahrweise (d.h. der Reaktor ist vollständig mit dem Reaktionsgemisch gefüllt) betriebenen 2 Liter-Edelstahldruckreaktor, in dem 200 g des Polyetherpolyols aus Beispiel 4 vorgelegt worden waren, wurden bei 130°C unter Rühren (800 U/min) über einen Zeitraum von 24 h folgende Komponenten mit den angegebenen Dosierraten dosiert:

- Propylenoxid mit 488 g/h
- Ethylenoxid mit 61 g/h
- Mischung aus 81 g Urethan-Alkohol aus Beispiel 1a und 32 mg DMC-Katalysator mit 81 g/h

Die Reaktionsmischung wurde über eine Produktaustragsleitung kontinuierlich aus dem Reaktor entnommen und zur Vervollständigung der Reaktion in einen auf 130°C temperierten 1 Liter-Rohrnachreaktor überführt. Aus dem erhaltenen Reaktionsgemisch wurden leicht flüchtige Anteile bei 130°C für 60 min unter vermindertem Druck bei 50 mbar (absolut) abdestilliert und anschließend auf Raumtemperatur abgekühlt.
Eine nach 24 h Reaktionszeit gezogene Probe wurde analysiert.

Produkteigenschaften:

| | |
|---|---|
| OH-Zahl: | 59,0 mg KOH/g |
| Viskosität (25°C): | 549 mPas |

**<u>Herstellung von Polyurethan-Weichschäumen:</u>**

**Beispiele 11 & 12: Polyetherpolyole aus Beispiel 8 ("P2P CAOS") und Beispiel 10 ("CAOS Conti")**

Es wurden Polyurethanschäume gemäß den in der nachfolgenden Tabelle angegebenen Rezepturen hergestellt. Aufgeführt sind die Anteile der Komponenten in Gewichts-Teilen. Es wurden qualitativ hochwertige Weichschaumstoffe mit einheitlicher Zellstruktur erhalten, die durch Ermittlung der Rohdichten und Stauchhärten charakterisiert wurden.

Tabelle 1: Herstellung von Polyurethan-Weichschaumstoffen

| **Beispiel** | **11a** | **11b** | **12a** | **12b** |
|---|---|---|---|---|
| Polyol aus Beispiel 8 | 100 | 100 | - | - |
| Polyol aus Beispiel 10 | - | - | 100 | 100 |
| | | | | |
| Stabilisator 1 | 2,4 | 1,2 | 2,4 | 1,2 |
| Katalysator 1 | 0,15 | 0,12 | 0,15 | 0,12 |
| Katalysator 2 | 0,14 | 0,18 | 0,14 | 0,18 |
| Wasser | 2,50 | 4,50 | 2,50 | 4,50 |
| Isocyanat 1 | 35,1 | 55,9 | 35,5 | 56,3 |
| NCO-Index | 108 | 108 | 108 | 108 |
| Rohdichte (kg/$m^3$) | 36,6 | 24,1 | 37,0 | 24,1 |
| Stauchhärte, 4.Zyklus (kPa) | 2,8 | 2,6 | 2,9 | 3,5 |

**Patentansprüche**

1. Verfahren zur Herstellung von Polyetherpolyolen durch Anlagerung von Alkylenoxiden an H-funktionelle Starterverbindungen, **dadurch gekennzeichnet, dass** mindestens ein Urethan-Alkohol gemäß Formel (II),

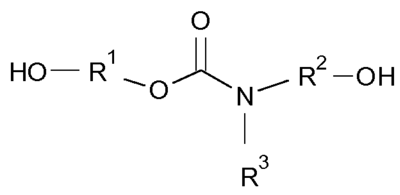

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann,
$R^2$ bedeutet lineares oder verzweigtes $C_2$ bis $C_{24}$ - Alkylen, das gegebenenfalls durch Heteroatome wie O, S oder N unterbrochen sein kann und substituiert sein kann,
$R^3$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können,

als H-funktionelle Starterverbindung eingesetzt wird.

**2.** Verfahren gemäß Anspruch 1, wobei
$R^1 = CH_2-CH_2$ oder $CH_2-CH(CH_3)$ bedeutet,
$R^2 = CH_2-CH_2$ oder $CH_2-CH(CH_3)$ bedeutet, und
$R^3$ = H bedeutet.

**3.** Verfahren gemäß Anspruch 1, **dadurch gekennzeichnet, dass** der Urethan-Alkohol erhalten wird durch Umsetzung von Propylencarbonat und/oder Ethylencarbonat mit einem Alkanolamin gemäß Formel (III),

$$HN(R^3)-R^2-OH \qquad (III)$$

wobei $R^2$ und $R^3$ die in Anspruch 1 genannte Bedeutung haben.

**4.** Verfahren gemäß Anspruch 3, **dadurch gekennzeichnet, dass** der Urethan-Alkohol erhalten wird durch Umsetzung von Propylencarbonat und/oder Ethylencarbonat mit mindestens einem Amin ausgewählt aus der Gruppe bestehend aus Ethanolamin, Diethanolamin, (N-Methyl)-Ethanolamin, Isopropanolamin, Diisopropanolamin und Propanolamin.

**5.** Verfahren gemäß einem der Ansprüche 1 bis 4, **dadurch gekennzeichnet, dass** als Alkylenoxid wenigstens ein Alkylenoxid ausgewählt aus der Gruppe bestehend aus Ethylenoxid und Propylenoxid eingesetzt wird.

**6.** Verfahren gemäß einem der Ansprüche 1 bis 5, wobei die Anlagerung in Gegenwart mindestens eines DMC-Katalysators erfolgt.

**7.** Verfahren gemäß Anspruch 6, wobei zunächst wenigstens ein Urethan-Alkohol der Formel (II) und der Doppelmetallcyanid-Katalysator vorgelegt werden und anschließend das Alkylenoxid hinzugefügt wird.

**8.** Verfahren gemäß einem der Ansprüche 1 bis 7, wobei eine oder mehrere der Urethan-Alkohole der Formel (II) als H-funktionelle Startersubstanz(en) während der Reaktion kontinuierlich in den Reaktor zudosiert werden.

**9.** Verfahren gemäß Anspruch 6, wobei ein H-funktionelles Starterpolyol **S-I** und der Doppelmetallcyanid-Katalysator vorgelegt werden und dann wenigstens ein Urethan-Alkohol der Formel (II) kontinuierlich gemeinsam mit einem oder mehreren Alkylenoxiden zudosiert werden, wobei das H-funktionelle Starterpolyol **S-I** eine OH-Zahl im Bereich von 3 mg KOH/g bis 1000 mg KOH/g aufweist, und wobei das resultierende Reaktionsgemisch kontinuierlich aus dem Reaktor nach einer vorwählbaren mittleren Verweilzeit entfernt wird.

**10.** Verfahren gemäß Anspruch 8 oder 9, wobei zusätzlich auch DMC-Katalysator kontinuierlich in den Reaktor dosiert wird und das resultierende Reaktionsgemisch kontinuierlich aus dem Reaktor entfernt wird.

**11.** Verfahren gemäß Anspruch 9 oder 10, wobei das kontinuierlich aus dem Reaktor entfernte Reaktionsgemisch mit einem Gehalt von 0,05 Gew.-% bis 10 Gew.-% Alkylenoxid in einen Nachreaktor überführt wird, in dem im Wege einer Nachreaktion der Gehalt an freiem Alkylenoxid auf weniger als 0,05 Gew.-% im Reaktionsgemisch reduziert wird.

**12.** Verfahren gemäß Anspruch 9, **dadurch gekennzeichnet, dass** als Starterpolyol S-I ein Polyetherpolyol enthaltend eine Struktureinheit der Formel (IV),

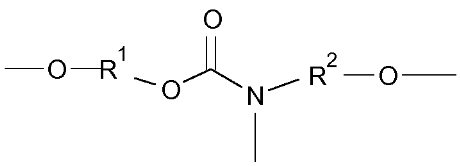

(IV)

wobei $R^1$ und $R^2$ die in Anspruch 1 oder 2 genannte Bedeutung haben und wobei $R^1$ und $R^2$ identisch oder voneinander verschieden sein können, oder
ein Polyetherpolyol erhältlich nach einem Verfahren gemäß einem der Ansprüche 1 bis 11 eingesetzt wird.

## Claims

**1.** Process for preparing polyether polyols by addition of alkylene oxides onto H-functional starter compounds, **characterized in that** at least one urethane alcohol of formula (II)

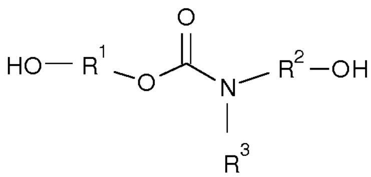

(II)

where

$R^1$ is linear or branched $C_2$- to $C_{24}$-alkylene which may optionally be interrupted by heteroatoms such as O, S or N and may be substituted,
$R^2$ is linear or branched $C_2$- to $C_{24}$-alkylene which may optionally be interrupted by heteroatoms such as O, S or N and may be substituted,
$R^3$ is H, linear or branched $C_1$- to $C_{24}$-alkyl, $C_3$- to $C_{24}$-cycloalkyl, $C_4$- to $C_{24}$-aryl, $C_5$- to $C_{24}$-aralkyl, $C_2$- to $C_{24}$-alkenyl, $C_2$- to $C_{24}$-alkynyl, each of which may optionally be interrupted by heteroatoms such as O, S or N and/or each of which may be substituted by alkyl, aryl and/or hydroxyl,

is used as H-functional starter compound.

**2.** Process according to Claim 1, wherein
$R^1 = CH_2\text{-}CH_2$ or $CH_2\text{-}CH(CH_3)$,
$R^2 = CH_2\text{-}CH_2$ or $CH_2\text{-}CH(CH_3)$, and
$R^3 = H$.

**3.** Process according to Claim 1, **characterized in that** the urethane alcohol is obtained by reacting propylene carbonate and/or ethylene carbonate with an alkanolamine of formula (III)

$$HN(R^3)\text{-}R^2\text{-}OH \qquad (III)$$

where $R^2$ and $R^3$ are as defined in claim 1.

**4.** Process according to Claim 3, **characterized in that** the urethane alcohol is obtained by reacting propylene carbonate

and/or ethylene carbonate with at least one amine selected from the group consisting of ethanolamine, diethanolamine, (N-methyl)ethanolamine, isopropanolamine, diisopropanolamine and propanolamine.

**5.** Process according to any of Claims 1 to 4, **characterized in that** the alkylene oxide used is at least one alkylene oxide selected from the group consisting of ethylene oxide and propylene oxide.

**6.** Process according to any of Claims 1 to 5, wherein the addition is effected in the presence of at least one DMC catalyst.

**7.** Process according to Claim 6, wherein at least one urethane alcohol of the formula (II) and the double metal cyanide catalyst are first initially charged and then the alkylene oxide is added.

**8.** Process according to any of Claims 1 to 7, wherein one or more urethane alcohols of the formula (II) are metered continuously into the reactor as H-functional starter substance(s) during the reaction.

**9.** Process according to Claim 6, wherein an H-functional starter polyol **S-I** and the double metal cyanide catalyst are initially charged and then at least one urethane alcohol of the formula (II) is metered in continuously together with one or more alkylene oxides, wherein the H-functional starter polyol **S-I** has an OH number in the range from 3 mg KOH/g to 1000 mg KOH/g, and wherein the resulting reaction mixture is removed continuously from the reactor after a pre-selectable mean residence time.

**10.** Process according to Claim 8 or 9, wherein DMC catalyst is additionally also metered continuously into the reactor and the resulting reaction mixture is removed continuously from the reactor.

**11.** Process according to Claim 9 or 10, wherein the reaction mixture removed continuously from the reactor with a content of 0.05% by weight to 10% by weight of alkylene oxide is transferred into a postreactor in which, by way of a postreaction, the content of free alkylene oxide is reduced to less than 0.05% by weight in the reaction mixture.

**12.** Process according to Claim 9, **characterized in that** the starter polyol S-I used is a polyether polyol containing a structural unit of the formula (IV)

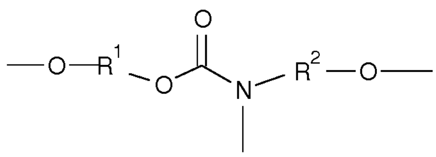

(IV)

where $R^1$ and $R^2$ are as defined in claim 1 or 2 and where $R^1$ and $R^2$ may be identical or different, or a polyether polyol obtainable by a process according to any of Claims 1 to 11.

## Revendications

**1.** Procédé de fabrication de polyéther-polyols par addition d'oxydes d'alkylène sur des composés de départ à fonction H, **caractérisé en ce qu'**au moins un uréthane-alcool selon la formule (II)

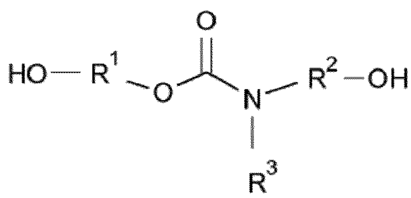

(II)

dans laquelle

$R^1$ signifie alkylène en $C_2$ à $C_{24}$ linéaire ou ramifié, qui peut éventuellement être interrompu par des hétéroatomes

tels qu'O, S ou N, et qui peut être substitué,
$R^2$ signifie alkylène en $C_2$ à $C_{24}$ linéaire ou ramifié, qui peut éventuellement être interrompu par des hétéroatomes tels qu'O, S ou N, et qui peut être substitué,
$R^3$ signifie H, alkyle en $C_1$ à $C_{24}$ linéaire ou ramifié, cycloalkyle en $C_3$ à $C_{24}$, aryle en $C_4$ à $C_{24}$, aralkyle en $C_5$ à $C_{24}$, alcényle en $C_2$ à $C_{24}$, alcynyle en $C_2$ à $C_{24}$, qui peuvent chacun éventuellement être interrompus par des hétéroatomes tels qu'O, S ou N et/ou qui peuvent chacun être substitués par alkyle, aryle et/ou hydroxyle,
est utilisé en tant que composé de départ à fonction H.

**2.** Procédé selon la revendication 1, dans lequel

$R^1$ signifie $CH_2$-$CH_2$ ou $CH_2$-$CH(CH_3)$,
$R^2$ signifie $CH_2$-$CH_2$ ou $CH_2$-$CH(CH_3)$,
$R^3$ signifie H.

**3.** Procédé selon la revendication 1, **caractérisé en ce que** l'uréthane-alcool est obtenu par mise en réaction de carbonate de propylène et/ou de carbonate d'éthylène avec une alcanolamine selon la formule (III)

$$HN(R^3)\text{-}R^2\text{-}OH \qquad (III)$$

dans laquelle $R^2$ et $R^3$ ont la signification indiquée dans la revendication 1.

**4.** Procédé selon la revendication 3, **caractérisé en ce que** l'uréthane-alcool est obtenu par mise en réaction de carbonate de propylène et/ou de carbonate d'éthylène avec une amine choisie dans le groupe constitué par l'éthanolamine, la diéthanolamine, la (N-méthyl)-éthanolamine, l'isopropanolamine, la diisopropanolamine et la propanolamine.

**5.** Procédé selon l'une quelconque des revendications 1 à 4, **caractérisé en ce qu'**au moins un oxyde d'alkylène choisi dans le groupe constitué par l'oxyde d'éthylène et l'oxyde de propylène est utilisé en tant qu'oxyde d'alkylène.

**6.** Procédé selon l'une quelconque des revendications 1 à 5, dans lequel l'addition a lieu en présence d'au moins un catalyseur DMC.

**7.** Procédé selon la revendication 6, dans lequel au moins un uréthane-alcool de formule (II) et le catalyseur de cyanure de métal double sont tout d'abord chargés, puis l'oxyde d'alkylène est ajouté.

**8.** Procédé selon l'une quelconque des revendications 1 à 7, dans lequel un ou plusieurs des uréthane-alcools de formule (II) en tant que substance(s) de départ à fonction H sont ajoutés en continu dans le réacteur pendant la réaction.

**9.** Procédé selon la revendication 6, dans lequel un polyol de départ à fonction H S-I et le catalyseur de cyanure de métal double sont chargés, puis au moins un uréthane-alcool de formule (II) est ajouté en continu conjointement avec un ou plusieurs oxydes d'alkylène, le polyol de départ à fonction H S-I présentant un indice OH dans la plage allant de 3 mg KOH/g à 1 000 mg KOH/g, et le mélange réactionnel résultant étant déchargé du réacteur en continu après un temps de séjour moyen pouvant être choisi au préalable.

**10.** Procédé selon la revendication 8 ou 9, dans lequel le catalyseur DMC est en outre également ajouté en continu dans le réacteur et le mélange réactionnel résultant est déchargé en continu du réacteur.

**11.** Procédé selon la revendication 9 ou 10, dans lequel le mélange réactionnel déchargé en continu du réacteur ayant une teneur de 0,05 % en poids à 10% en poids d'oxyde d'alkylène est transféré dans un réacteur secondaire, dans lequel la teneur en oxyde d'alkylène libre est réduite à moins de 0,05 % en poids dans le mélange réactionnel au cours d'une réaction secondaire.

**12.** Procédé selon la revendication 9, dans lequel un polyéther-polyol contenant une unité structurale de formule (IV)

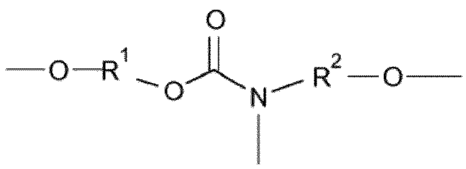

(IV)

dans laquelle $R^1$ et $R^2$ ont la signification indiquée dans la revendication 1 ou 2 et dans laquelle $R^1$ et $R^2$ peuvent être identiques ou différents l'un de l'autre, ou
un polyéther-polyol pouvant être obtenu par un procédé selon l'une quelconque des revendications 1 à 11 est utilisé en tant que polyol de départ S-I.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3829505 A
- DE 1595759
- US 3654224 A
- US 5001210 A
- EP 1359177 A
- US 3404109 A
- US 3941849 A
- US 5158922 A
- US 5470813 A
- EP 0700949 A
- EP 0743093 A
- EP 0761708 A
- WO 9740086 A
- WO 9816310 A
- WO 0047649 A
- WO 2011144523 A
- JP 4145123 A
- WO 0139883 A
- WO 0180994 A
- US 4987271 A
- DE 3132258 A
- EP 0406440 A
- US 5391722 A
- US 5099075 A
- US 4721818 A
- US 4877906 A
- EP 0385619 A
- US 3538043 A
- US 4500704 A
- US 5032671 A
- US 6646100 A
- EP 0222453 A
- WO 2008013731 A
- WO 9914258 A
- EP 1577334 A
- WO 9729146 A
- WO 9803571 A
- EP 0007502 A1


### In der Beschreibung aufgeführte Nicht-Patentliteratur


- **INOUE et al.** Copolymerization of Carbon Dioxide and Epoxide with Organometallic Compounds. *Die Makromolekulare Chemie,* 1969, vol. 130, 210-220
- Ullmann's Encyclopedia of Industrial Chemistry. 1992, vol. B4, 167ff
- Handbuch Apparate. Vulkan-Verlag Essen, 1990, 188-208
- **W. SIEFKEN.** *Justus Liebigs Annalen der Chemie,* vol. 562, 75-136